# EUROPEAN PATENT SPECIFICATION

(11) **EP 0 510 771 B1**
(45) Date of publication and mention of the grant of the patent: **24.07.1996**
(21) Application number: 92201129.1
(22) Date of filing: 21.04.1992
(51) Int. Cl.: B01J 37/00, B01J 23/74, C07C 1/04, C10G 2/00

(54) **Process for the preparation of an extruded silica-based catalyst of catalyst precursor**
Verfahren zur Herstellung eines extrudierten Silika Katalysators oder katalysatorvorläufers
Procédé pour préparer un catalyseur ou un précurseur de catalyseur extrudé à base de silice

(30) Priority: 23.04.1991 GB 9108663
(43) Date of publication of application: 28.10.1992
(73) Proprietor: SHELL INTERNATIONALE RESEARCH MAATSCHAPPIJ B.V., NL-2596 HR Den Haag (NL)
(72) Inventor: Reinalda, Donald, NL-1031 CM Amsterdam (NL); Blankenstein, Paul, NL-1031 CM Amsterdam (NL); Derking, Anke, NL-1031 CM Amsterdam (NL)

(56) References cited:
- EP-A- 0 309 048
- EP-A- 0 428 223
- EP-A- 0 455 307
- GB-A- 2 125 062
- US-A- 5 073 661

## Description

The present invention relates to a process for the preparation of a catalyst or catalyst precursor, in particular to a process for the preparation of a catalyst or catalyst precursor comprising a catalytically active component selected from elements in Group VIII of the Periodic Table, preferably cobalt, and a promoter selected from the elements in Group IVB of the Periodic Table.

The preparation of hydrocarbons from a gaseous mixture comprising carbon monoxide and hydrogen by contacting the mixture with a catalyst at elevated temperature and pressure is known in the literature as the Fischer-Tropsch synthesis.

Catalysts used in the Fischer-Tropsch synthesis often comprise one or more metals from Group VIII of the Periodic Table of Elements, especially from the iron group, optionally in combination with one or more metal oxides and/or other metals as promoters. Recently, particular interest has been given to catalysts comprising cobalt as the catalytically active component, in combination with one or more promoters selected from the elements of Group IVB of the Periodic Table, and supported on a silica carrier. Such catalysts are known in the art and have been described, for example, in the specification of European patent application publication No. EP-A-127 220.

UK patent application publication No. GB-2 125 062 describes a process for the preparation of a catalyst in which silica is kneaded with a solvent and a cobalt compound. The resulting mixture is then dried and calcined, to yield the finished catalyst.

Typically, the catalysts of the prior art have been prepared in the form of silica spheres. However, the use of spherical catalyst particles in a commercial scale reaction vessel may lead to a very high pressure drop across a fixed bed containing the particles, to problems associated with the maldistribution of the catalytically active component through the fixed bed and to problems associated with heat transfer from the fixed bed. It has been found that such problems may be obviated by the use of elongate, extruded catalyst particles, in particular in the shape of polylobes, especially trilobes.

Accordingly, there exists a need for a process for preparing an extruded silica-based catalyst comprising a catalytically active metal component selected from elements in Group VIII of the Periodic Table, preferably cobalt, and a promoter selected from the elements of Group IVB of the Periodic Table easily and efficiently in sufficient quantities to meet the demands of commercial scale Fischer-Tropsch reaction vessels.

European patent application publication No. 0 167 324 (EP-A-0 167 324) discloses a method for extruding silica-rich solids comprising mixing the silica-rich solids with water and an alkali metal compound, mulling the mixture, extruding the mixture and subsequently drying the extrudates. However, the inclusion of alkali metal compounds in the mixture has been found to be undesirable as the alkali metal ions can severely impair the catalytic behaviour of the eventual catalyst. The alkali metal ions may be removed from the extrudates of EP-A-0 167 324 by soaking in a solution of ammonium nitrate and nitric acid. However, the need for such a step is undesirable in the preparation of a commercial catalyst. Accordingly, a method for extruding silica which is not reliant on the presence of alkali metal compounds in the extrusion mixture is required.

European patent application publication No. 0 309 048 (EP-A-0 309 048) discloses a process for the preparation of a shapable dough comprising mixing and kneading a particulate silica with water and ammonia or an ammonia-releasing agent to obtain a mixture having a total solids content of from 25 to 60% by weight, the ammonia being present in an amount of from 0.5 to 20% by weight on the total solids content of the mixture. The dough may then be extruded to form silica extrudates. The surface area, without the presence of catalytically active metals, is typically in the range from 66 to 220 m²/g. It is mentioned in EP-A-0 309 048 that it is possible, prior to extrusion, to admix titanium dioxide or zirconium dioxide with the dough. This is said to be advantageous when the silica is used as carrier and the titanium and zirconium are the effective catalyst ingredients.

European patent application No. 0 313 163 (EP-A-0 313 163) discloses a process for the preparation of a shapable dough which comprises mixing and kneading a particulate silica-alumina with water and with an alkanolamine or ammonia to obtain a mixture having a total solids content of from 25 to 60% by weight, the alkanolamine or ammonia being present in an amount of from 0.5 to 20% by weight of the total solids content of the mixture. The dough may be extruded to form silica-alumina extrudates. EP-A-0 313 163 mentions that titanium dioxide or zirconium dioxide may be admixed with the dough prior to extrusion.

Finally, European patent application No. 90.202989.1 (published as EP-A-0 428 223) describes a process for the preparation of extrudates suitable for use in the manufacture of catalysts or catalyst carriers, comprising mulling a mixture of finely divided silica, a water soluble compound derived from a metal selected from Group IVB of the Periodic Table and water, which mixture has a solids content of from 20 to 50% by weight, and extruding the mixture. The resulting extrudates may be impregnated with a suitable catalytically active metal, for example cobalt.

Surprisingly, a novel process has been found, which allows the preparation of a catalyst or catalyst precursor comprising a catalytically active metal component selected from elements in Group VIII of the Periodic Table, preferably cobalt, and a promoter selected from the elements of Group IVB of the Periodic Table, which catalyst and catalyst precursor are of high quality and possess desirable properties.

Accordingly, in one embodiment, the present invention provides a process for the preparation of silica-based extrudates for use as a Fischer-Tropsch catalyst or precursor therefor and comprising a catalytically active component selected from elements in Group VIII of the Periodic Table and a promoter selected from elements in Group IVB of the Periodic Table, which process comprises mulling a mixture comprising silica, a solvent and a source of said catalytically active component, which mixture has a solids content of from 20 to 60 %wt; extruding the mulled mixture; and depositing on the resulting extrudates a source of said promotor.

In another embodiment, the present invention relates to a process for the preparation of silica-based extrudates for use as a Fischer-Tropsch catalyst or precursor therefor and comprising a catalytically active component selected from elements in Group VIII of the Periodic Table and a promoter selected from elements in Group IVB of the Periodic Table, which process comprises mulling a mixture comprising silica, a solvent and a source of said promoter, which mixture has a solids content of from 20 to 60 %wt; extruding the mulled mixture; and depositing on the resulting extrudates a source of said catalytically active component; with the proviso that the source of the promoter is insoluble in the solvent.

In yet another embodiment, the present invention relates to a process for the preparation of silica-based extrudates for use as a Fischer-Tropsch catalyst or precursor therefor and comprising a catalytically active component selected from elements in Group VIII of the Periodic Table and a promoter selected from elements in Group IVB of the Periodic Table, which process comprises mulling a mixture comprising silica, a solvent, a source of said catalytically active component and a source of said promoter, which mixture has a solids content of from 20 to 60 %wt; extruding the mulled mixture.

The silica used in the process of the present invention is preferably a finely divided silica and preferably comprises silica particles having an average diameter which is less than 100 µm, preferably between 15 and 80 µm, more preferably between 35 and 65 µm.

A silica which may be used in the process of the invention is often indicated as amorphous silica, and is usually a porous silica. The word amorphous, when used in combination with silica, denotes a lack of crystal structure, as defined by X-ray diffraction. Some short-range ordering of the silica may be present and is indicated by electron diffraction studies, but this ordering gives no sharp X-ray diffraction pattern. The extent of porosity may be indicated by the pore volume and/or the surface area.

A very suitable silica for use in the process of the invention is silica gel, a more or less coherent, rigid, continuous three-dimensional network of particles of colloidal silica. The amount of silicon dioxide is typically from 96 to 99.5% by weight. The aggregate particle size is typically from 3 to 25 µm, while the ultimate particle size is typically from 1 to 100 nm. The surface area may vary from 150 to 900 m²/g and is often in the range of from 200 to 700 m²/g. Especially suitable silica gels are spray dried silica gels. It is preferred not to use calcined silica gels, that is silica gels which have been heated to temperatures around 500 °C and higher.

A preferred silica for use in the process of the present invention is precipitated silica. Precipitated silica is composed of aggregates of ultimate particles of colloidal size that have not become linked in a massive gel network during the preparation process. The amount of silicon dioxide is typically from 80 to 99.5 % by weight. The aggregate particle size is typically from 3 to 65 µm, while the ultimate particle size is typically from 3 to 30 nm. The surface area may vary from 30 to 900 m²/g, and is typically from 45 to 700 m²/g.

Precipitated silica may be prepared from a silicate solution, preferably a sodium or potassium silicate, by addition of an acid, preferably sulphuric acid or hydrochloric acid. The precipitates are separated from the mother liquor by filtration. It is especially preferred in the process of the present invention to use the filter cake which is obtained after filtration of the product as described above, more preferably the washed and/or spray dried filter cake. Washing may be carried out with water, but is preferably carried out with an electrolyte solution having a pH lower than 6. An organic acid, for instance acetic acid, or an inorganic acid, for instance hydrogen fluoride or nitric acid, or salts thereof may be used. Washing may also be carried out after spray drying of the filter cake.

Alternatively, pyrogenic or fumed silica may be used in the process of the present invention. This type of silica is usually obtained in high temperature processes by vaporising silica, usually sand, at 2000 °C and cooling, thus forming anhydrous amorphous silica particles. Other processes for the preparation of pyrogenic or fumed silica include the oxidation of silicon tetrachloride vapour with oxygen or with hydrogen and/or methane and the flame hydrolysis of silicon ester vapours. Pyrogenic silica tends to be less dense than other types of silica. The amount of silicon dioxide is typically greater than 99.5% by weight. The aggregate particle size is typically from 1 to 10 µm, often from 2 to 5 µm, while the ultimate particle size is typically from 1 to 100 nm. The surface area may vary from 10 to 500 m²/g, and is often from 15 to 400 m²/g.

The purity of the silica to be used in the process of the present invention is preferably greater than 97% by weight based on water free samples, more preferably greater than 98%, especially greater than 99%. It is preferred to use a silica which contains an amout of sodium which is less than 10,000 ppmw, more preferably less than 6,000 ppmw, still more preferably less than 2,000 ppmw. The amount of sulphate present in the silica is preferably less than 7,500 ppmw, more preferably less than 4,500 ppmw, still more preferably less than 1500 ppmw.

The silica to be used in the process of the present invention may be washed before use in order to increase its purity. Water or an electrolyte solution may be used. The washing solution preferably has a pH lower than 6. Suitable washing solutions include aqueous solutions of organic acids, for example alkanoic acids having 1 to 12 carbon atoms, preferably 1 to 6 carbon atoms, and dicarboxylic acids, preferably containing 1 to 6 carbon atoms. Preferably alkanoic acids such as formic acid, acetic acid, propionic acid and butyric acid are used in the washing. Acetic acid is especially preferred. Alternatively, washing solutions comprising inorganic acids, such as hydrogen fluoride, hydrogen chloride, hydrogen bromide, nitric acid, nitrous acid and perchloric acid may be used. Further, salts of the aforementioned acids may be used, for example ammonium salts, or mixtures of the aforementioned acids and one or more salts thereof.

The solids content of the mixture to be mulled in the process of the present invention is from 20 to 60% by weight, preferably 30 to 50% by weight, more preferably about 40%. The amount of solvent present should at least be such that a mixture is formed having a consistency suitable for extrusion.

The mixture to be mulled comprises a solvent. The solvent may be any suitable solvent known in the art, for example water; alcohols, such as methanol, ethanol and propanol; ketones, such as acetone; aldehydes, such as propanal; and aromatic solvents, such as toluene. The solvent is most preferably water.

The catalyst or catalyst precursor comprises a catalytically active component selected from elements in Group VIII and a promoter selected from elements in Group IVB of the Periodic Table. Preferred catalytically active components are the elements iron, nickel and cobalt, with cobalt being especially preferred. Elements in Group IVB suitable for use as promoters include titanium, zirconium and hafnium. The promoter is preferably zirconium or titanium, with zirconium being especially preferred.

The mixture comprises one or both of a source of the catalytically active component and a source of the promoter. Suitable sources of the catalytically active component for inclusion in the mixture include compounds which are both soluble and insoluble in the solvent. Suitable sources include salts derived from organic acids, for example acetates, benzoates and propionates; halides, for example chlorides, bromides, iodides and fluorides; and other salts, for example nitrates, oxides, hydroxides, carbonates and chlorates. Preferably, the source is substantially insoluble in the solvent. The source is preferably hydroxide, with cobalt hydroxide being especially preferred.

Suitable sources for the promoter include compounds of the elements in Group IVB which are both soluble and insoluble in the solvent. Thus, suitable promoter sources include salts derived from organic acids, for example acetates, benzoates and propionates; halides, for example fluorides, chlorides, bromides and iodides; and other salts, for example nitrates, oxides, hydroxides, carbonates and chlorates. In cases in which the mixture does not comprise a source for the catalytically active component, the promoter source is insoluble in the solvent. In such cases, the promoter source is preferably a hydroxide. In cases in which the mixture comprises a source for the catalytically active component, the promoter source may be either soluble or insoluble in the solvent, a most convenient source being the hydroxide.

To obtain strong extrudates, it is preferred to include in the mixture a basic compound to act as a peptizing agent for the silica. The basic compound is preferably ammonia, an ammonia releasing compound, an ammonium compound or an organic amine. Such basic compounds are removed upon calcination and are not retained in the extrudates to impair the catalytic performance of the final product. The basic compound is most preferably an organic amine or an ammonium compound. A most suitable organic amine is ethanol amine. It has been found convenient to include in the mixture a compound of the required element of Group IVB to act as both promoter source and as the basic compound. One such preferred group of compounds are ammonium carbonates of the Group IVB elements. Ammonium zirconium carbonate is particularly preferred.

A basic compound of the elements of Group IVB may be used in addition to both one or more of the aforementioned promoter sources and a basic compound. However, it has been found possible to dispense with both an additional promoter source and an additional basic compound by using a basic compound of the elements of Group IVB.

The amount of basic compound included in the mixture should be sufficient to peptise the silica present in the mixture. The amount of basic compound present in the mixture can be readily determined by measuring the pH of the mixture. During mulling the mixture should preferably have a pH in the range of from 8.5 to 11.5, preferably from 9.0 to 11.0.

It has been found that the extrudates produced by the extrusion of mixtures having a high pH may be less satisfactory, owing to the continued action of the peptising agent, than those produced by the extrusion of mixtures having a pH in the range of from 7.0 to 8.5. Accordingly, to avoid extruding a mixture having a high pH, it is preferred to reduce the pH of the mixture, after allowing sufficient time for the silica to be peptised by the basic compound, but before extrusion, to a pH value in the range of from 7.0 to 8.5. The reduction in pH may be effected by the addition of an acid. Suitable acids include both organic and inorganic acids. Suitable inorganic acids include aqueous solutions of hydrogen fluoride, hydrogen chloride and hydrogen bromide, nitric acid, nitrates acid and perchloric acid. Preferably, an organic acid is used, for example alkanoic acids having from 1 to 12 carbon atoms, preferably from 1 to 6 carbon atoms, or dicarboxylic acids having from 1 to 6 carbon atoms. Particularly preferred acids are the alkanoic acids, especially formic acid, acetic acid, propionic acid and butanoic acid. Acetic acid is most preferred.

To improve the flux properties of the mixture during extrusion a surface active agent or polyelectrolyte may be added to the mixture. The addition of the surface active agent further results in a smoother extrudate texture and facilitates cutting of the extruded product. Further, formation of pores in the calcined catalytic material may be improved which may enhance the catalytic properties of the products. Suitable surface active agents include cationic surface active agents, for example fatty amines, quaternary ammonium compounds, aliphatic monocarboxylic acids, ethoxylated alkyl amines, polyvinyl pyridine, sulphoxonium, sulphonium, phosphonium and iodonium compounds; anionic surface active agents, for example alkylated aromatic compounds, acyclic monocarboxylic acids, fatty acids, sulphonated aromatic compounds, alcohol sulphates, ether alcohol sulphates, sulphated fats and oils and phosphonic acid salts; and nonionic surface active agents, for example polyethylene alkylphenols, polyoxyethylene alcohols, polyoxyethylene alkylamines, polyoxyethylene alkylamides, polyols and acetylenic glycols. The amount of surface active agent is typically from 2 to 8% by weight, preferably from 3 to 5% by weight, based on the weight of silica carrier in the mixture. A preferred surface active agent is sold under the trademark Nalco. The surface active agent may be added at any stage during the mulling of the mixture prior to extrusion, preferably just prior to extrusion.

It is possible prior to extrusion to a admix with the mixture titanium dioxide, zirconium dioxide and/or aluminium trioxide, or precursor compounds therefor such as hydroxides of titanium, zirconium or aluminium. Other admixtures that may be used are for instance oxides of gallium, indium, thorium, uranium, magnesium and zinc. The amount of each of the added compounds as indicated above is typically up to 20% by weight calculated on the amount of silica carrier, preferably up to 10%, more preferably up to 5%. The total amount most suitably does not exceed 50% by weight calculated on the amount of silica carrier, preferably does not exceed 30%, more preferably does not exceed 15% by weight.

In the process of the present invention, a mixture of the aforementioned components is mulled. In principle, it is possible to combine the components of the mixture in any order. However, it has been found advantageous to prepare and mull the mixture in the following manner. At the very least, the mixture comprises silica and a solvent, which are first mixed together. If the mixture is to include a basic compound, it has been found advantageous to add the basic compound to the mixture after the silica and solvent have been combined. In this way, the considerable take up of the basic compound into the pores of the silica is avoided, resulting in improved extrudates. Basic compound confined within the pores of the silica is prevented from fully peptising the silica, requiring the addition of further amounts of the basic compound for satisfactory peptisation, or yielding extrudates having a lower crush strength. The resulting mixture of silica, water and basic compound may be mixed or mulled. Thereafter, once the silica has been satisfactorily peptised, the pH of the mixture may be reduced as hereinbefore described. A source of the catalytically active component and/or a source of the promoter is added and the resulting mixture subjected to further mulling. A surface active agent, if desired, may be added at any time during the mulling, preferably just prior to a final period of mulling.

Typically, the mixture is mulled for a period of from 10 to 120 minutes, preferably from 15 to 90 minutes. During the mulling process, energy is input into the mixture by the mulling apparatus. The rate of energy input into the mixture is typically from 0.05 to 50 Wh/min/kg, preferably from 0.5 to 10 Wh/min/kg. The mulling process may be carried out over a broad range of temperature, preferably from 15 to 50 °C. As a result of the energy input into the mixture during the mulling process, there will be a rise in temperature of the mixture during the mulling. The mulling process is conveniently carried out at ambient pressure. Any suitable, commercially available mulling machine may be employed.

Once the mulling process has been completed, the resulting mixture is then extruded. Extrusion may be effected using any conventional, commercially available extruder. In particular, a screw-type extruding machine may be used to force the mixture through orifices in a suitable dieplate to yield extrudates of the desired form. The strands formed upon extrusion may be cut to the desired length.

The extrudates may have the form of cylinders, for example, a hollow cylinder, or may have a form which is multilobed or twisted multilobed in cross section, or take any other form known in the art. The process of the present invention has been found to be particularly suitable for forming trilobe extrudates. Typically, the extrudates have a nominal diameter of from 0.5 to 5 mm, preferably from 1 to 3 mm.

After extrusion, the extrudates are dried. Drying may be effected at an elevated temperature, preferably up to 800 °C, more preferably up to 300 °C. The period for drying is typically up to 5 hours, preferably from 30 minutes to 3 hours.

Preferably, the extrudates are calcined after drying. Calcination is effected at an elevated temperature, preferably up to 1000 °C, more preferably from 200 °C to 1000 °C most preferably from 300 °C to 800 °C. Calcination of the extrudates is typically effected for a period of up to 5 hours, preferably from 30 minutes to 4 hours.

The calcination may be effected by heating the extrudates in air. Alternatively, calcination may be by means of direct heating using the hot exhaust gases of a flame to contact the extrudates.

Typically, the pore volume of the calcined silica extrudates is between 0.8 and 1.5 ml/g, preferably between 1.15 and 1.35 ml/g. A typical surface area is between 100 and 500 m²/g, preferably between 200 and 400 m²/g. Typical pore-diameters are between 8 and 50 nm, preferably between 12 and 30 nm.

In the process of the present invention, the extrudates are then subjected to a deposition stage in which a source of the catalytically active component and a source of the promoter may be deposited on the finished extrudates. The original mixture used in the process comprised either or both a source of the catalytically active component or a source of the promoter. The deposition of a source for the said component or promoter already present in the mixture is optional. The addition to the extrudate by deposition of an additional source for either the catalytically active component or the promoter may be used to further increase the loading of the catalytically active component or the promoter in the final catalyst or catalyst precursor.

Deposition of a source of the catalytically active component or a source of the promoter on the extrudates may be effected by any of the techniques known in the art, for example kneading or precipitation. If deposition of both a source of the catalytically active component and a source of the promoter on the extrudate is to be effected, the deposition may be carried out in either one or two stages. The order of deposition of the catalytically active component source and the promoter source is largely a matter of choice and convenience. However, the preferred order is first to deposit a source for the promoter on the extrudates and secondly to deposit a source for the catalytically active component.

A preferred technique for the deposition is impregnation. Impregnation may be effected by contacting the extrudates with a compound of the catalytically active component or the promoter in the presence of a liquid, preferably in the form of a solution of the compound. Suitable liquids for use in the impregnation include both organic and inorganic liquids, water being a most convenient and preferred liquid. Suitable compounds of the catalytically active component and the promoter include both organic and inorganic compounds, with a preference for compounds which are soluble in the selected solvent. Preferably, the compounds are inorganic compounds. Nitrates are most preferred compounds.

The extrudates are most conveniently contacted with the compound of the catalytically active component or promoter compound by immersion in the liquid. Preferably, the extrudates are immersed in a sufficient volume of liquid so as to just fill the volume of pores in the extrudates.

If the impregnation is conducted in a single stage, the extrudates are contacted simultaneously with both a compound of the catalytically active component and a compound of the promoter in the presence of the liquid. Preferably, the extrudates are immersed in an aqueous solution of both a nitrate of the catalytically active component and a nitrate of the promoter. If the impregnation is conducted in two stages, the extrudates are contacted in a first stage with one of a compound of the catalytically active component and a compound of the promoter in the presence of a liquid and in a second stage with the other of a compound of the catalytically active component and a compound of the promoter in the presence of a liquid. The liquid may be the same or different in the two stages; most conveniently the same.

After the impregnation, if in a single stage, or after each stage in a two-stage impregnation, the extrudates are dried. The conditions under which the extrudates are dried are those as hereinbefore described. Preferably, after the or each drying process, the extrudates are calcined, the calcination conditions being those as hereinbefore described.

The product of the process of the present invention preferably comprises the catalytically active component in an amount of from 3 to 100 parts by weight, most preferably from 10 to 80 parts by weight, per 100 parts by weight of silica. The promoter is preferably present in an amount of from 1 to 60 parts by weight, more preferably from 2 to 40 parts by weight, per 100 parts by weight of silica.

The products of the process of the present invention find use in any process in which a promoted catalyst having a catalytically active component as hereinbefore defined is required. In particular, after undergoing a suitable reduction treatment, the product may be used a catalyst in a process for the preparation of hydrocarbons from a mixture of carbon monoxide and hydrogen; the so-called Fischer-Tropsch synthesis.

The products of the process of this invention may be reduced by contact with a hydrogen-containing gas at elevated temperature and pressure.

Thereafter, the resulting catalyst may be contacted with a mixture of carbon monoxide and hydrogen at an elevated temperature and pressure. Typically, the reaction is effected at a temperature in the range of from 125 to 350 °C, preferably from 175 to 250 °C. The reaction pressure is typically in a range of from 5 to 100 bar, preferably from 12 to 50 bar. The hydrogen/carbon monoxide molar ratio in the feed gas is typically greater than 1.5, preferably from 1.75 to 2.25. Unconverted hydrogen and carbon monoxide may be recycled to again contact the catalyst. In such an arrangement, the molar ratio of hydrogen to carbon monoxide in the gas actually contacting the catalyst may be considerably lower than that of the feed gas, typically in the range of from 0.9 to 1.2. The process of the present invention is further described in the following illustrative examples. In the examples, values for the loss on ignition are quoted on the basis of water lost upon heating the sample to a temperature in the range of from 550 to 600 °C. The extrusion process in the following examples was conducted using a 1" Bonnot extruder having a 1.7 mm Delrin trilobe matrix dieplate insert yielding straight trilobe extrudates having a nominal diameter of 1.7mm. Calcination was conducted at the temperature and for the duration quoted in an atmosphere of air.

### Example 1 (comparative)

### Extrudate Preparation

A mixture of silica (precipitated silica, average particle size 50 µm, surface area 450 m²/g, 75g on dry basis), monoethanolamine (3.8g as a 5% aqueous solution) and water (116g) was mulled for a period of 30 minutes. Water (6g) was added and the resulting mixture mulled for a further 20 minutes. Polyelectrolyte (Nalco) (1.5g as a 2% aqueous solution) was added and the resulting mixture mulled for a further 5 minutes to give a final mixture having a loss an ignition of 65% and a pH of 9.1. The final mixture was extruded using a 1" Bonnot extruder having a 1.7mm Delrin trilobe matrix dieplate insert to yield trilobe extrudates. The extrudates were dried at a temperature of 120 °C and calcined at a temperature of 530 °C, followed by a further calcination at 800 °C. The resulting extrudates, hereafter extrudates "A" had a high crush strength.

### Catalyst Preparation

An aqueous solution of zirconium nitrate (127 g/l Zr) was prepared by dissolving zirconyl nitrate (ZrO(NO₃)₂.xH₂O, 10.9g) in water to a total volume of 28.4 ml. The extrudates A were dried at a temperature of 300 °C. To impregnate the extrudates with zirconium, the extrudates A (29.93g) were mixed with the zirconium nitrate solution on a roller bank for 1 hour. The zirconium-impregnated extrudates were then dried for 1 hour in air at a temperature of 60 °C and then calcined at a temperature of 500 °C. An aqueous solution of cobalt nitrate (280 g/l Co) was prepared by dissolving cobalt nitrate (Co(NO₃)₂.6H₂O, 40.04g) in water to a total volume of 29 ml. The zirconium-impregnated extrudates (33.67g) were impregnated with cobalt by mixing with the cobalt nitrate solution. The cobalt/zirconium-impregnated extrudates were then heated with further mixing to a temperature of 120 °C, dried for 1 hour in air at a temperature of 60 °C and calcined at a temperature of 500 °C. The resulting cobalt/zirconium-impregnated extrudates are hereafter referred to as catalyst "A".

### Example 2 (comparative)

### Catalyst Preparation

Using the Extrudates A prepared in Example 1, a catalyst, hereafter catalyst "B", was prepared by the general procedure set out in Example 1, with the exception that an aqueous solution of zirconium nitrate comprising 20.8 g/l Zr was prepared by dissolving zirconyl nitrate (1.81g) in water to a total volume of 28.9 ml, which solution was then used to impregnate extrudates A with zirconium. The zirconium-impregnated extrudates were then dried as in Example 1 and used directly in the second impregnation as in Example 1, but without calcination.

### Example 3 (comparative)

### Catalyst Preparation

Extrudates A (29.95g) prepared in Example 1 were impregnated with cobalt by mixing with 46.7g of an aqueous solution (300 g/l Co) prepared by dissolving cobalt nitrate (Co(NO₃)₂.6H₂O, 100.0_{g}) in water to a total weight of 112.5 g. The cobalt-impregnated extrudates were further mixed at a temperature of 140 °C, dried for 1 hour in air at a temperature of 60 °C, and calcined at a temperature of 500 °C. An aqueous solution of zirconium nitrate (24.6 g/l Zr) was prepared by dissolving zirconyl nitrate (ZrO(NO₃)₂.xH₂O, 1.73g) in water to a total volume of 23.14 ml. The cobalt-impregnated extrudates were mixed with the zirconium nitrate solution. The resulting zirconium/cobalt-impregnated extrudates were further mixed at a temperature of 120 °C, dried for 1 hour in air at a temperature of 60 °C, and calcined at a temperature of 500 °C. The resulting zirconium/cobalt-impregnated extrudates are hereafter referred to as catalyst "C".

### Example 4 (comparative)

### Catalyst Preparation

An aqueous solution (280 g/l Co; 20 g/l Zr) was prepared by dissolving zirconyl nitrate (ZrO(NO₃)₂.xH₂O, 1.81g) and cobalt nitrate (Co(NO₃)₂.6H₂O, 41.46g) in water to a total volume of 30 ml. Extrudates A prepared in Example 1 (30g) were mixed with the aqueous solution to effect impregnation of the extrudates by cobalt and zirconium. The resulting impregnated extrudates were further mixed at a temperature of 120 °C, dried for 1 hour in air at a temperature of 60 °C and calcined at a temperature of 500 °C. The resulting impregnated extrudates are hereafter referred to as catalyst "D".

### Example 5

### Extrudate Preparation

A mixture of silica (precipitated silica, average particle size 50 µm, surface area 450 m²/g 75g on dry basis), monoethanolamine (3.8g as a 5% aqueous solution) and water (150 g) was mulled for a period of 10 minutes. Zirconium hydroxide (12.2g on basis of ZrO₂) was added and the resulting mixture mulled for a further 25 minutes. Polyelectrolyte (Nalco) (1.7g as a 2% aqueous solution) was added and the mixture mulled for a further 15 minutes to give a final plastic mixture having a loss on ignition of 66.2% and a pH of 8.7. The final mixture was extruded using a 1" Bonnot extruder having 1.7 mm Delrin trilobe matrix dieplate insert to yield trilobe extrudates. The extrudates were dried at a temperature of 120 °C, calcined for 2 hours at a temperature of 530 °C. The resulting extrudates, hereafter extrudates "B", had a high crush strength. A portion of the extrudates "B" were then further calcined at a temperature of 800 °C for 2 hours to yield extrudates "C".

### Catalyst Preparation

An aqueous solution (213 g/l Co) was prepared by dissolving cobalt nitrate (Co(NO₃)₂.6H₂O, 100.0g) in water to a total weight of 112.5g, from which a portion (36.14g) was removed and diluted with water to a volume of 30.5 ml. Extrudates B (27.0g) were mixed with the diluted solution to effect impregnation of the extrudates by cobalt. The cobalt-impregnated extrudates were further mixed at a temperature of 120 °C, dried in air at a temperature of 60 °C, and calcined at a temperature of 500 °C. The resulting cobalt-impregnated extrudates are hereafter referred to as catalyst "E".

### Example 6

### Catalyst Preparation

An aqueous solution (241 g/l Co) was prepared by dissolving cobalt nitrate (Co(NO₃)₂.6H₂O, 100g) in water to a total weight of 112.5g, from which a portion (29.15g) was removed and diluted with water to a volume of 21.74 ml. Extrudates C prepared in Example 5 (21.74g) were mixed with the diluted solution to effect impregnation of the extrudates with cobalt. The resulting cobalt-impregnated extrudates were further mixed at a temperature of 120 °C, dried in air for 1 hour at a temperature of 60 °C,and calcined at a temperature of 500 °C. The resulting cobalt-impregnated extrudates are hereafter referred to as catalyst "F".

### Example 7

### Extrudate Preparation

A mixture of silica (precipitated silica, average particle size 50 µm, surface area 450 m²/g, 75g on a dry basis), monoethanolamine (3.8g as a 5% aqueous solution) and water (150 g) was mulled for a period of 10 minutes. Cobalt hydroxide (28.6g on basis of Co₃O₄) was added and the resulting mixture mulled for a further 30 minutes. Polyelectrolyte (Nalco) (2.1g as a 2% aqueous solution) was added and the mixture mulled for further 5 minutes to give a final mixture having a loss on ignition of 61.1% and a pH of 9.1. The final mixture was extruded using a 1" Bonnot extruder having a 1.7 mm Delrin trilobe dieplate insert to yield trilobe extrudates. The extrudates were dried at a temperature of 120 °C and calcined at a temperature of 530 °C for 2 hours. The resulting extrudates, hereafter designated extrudates "D", had a high crush strength.

### Catalyst Preparation

An aqueous solution (14.4 g/l Zr) was prepared by dissolving zirconyl nitrate (ZrO(NO₃)₂.xH₂O, 1.31g) in water to a total volume of 29.79 ml. Extrudates D (30.01g) were immersed in the aqueous solution to effect impregnation of the extrudates with cobalt. The resulting cobalt-impregnated extrudates were then dried and finally calcined at 500 °C for 1 hour. The resulting cobalt-impregnated extrudates are hereafter referred to as catalyst "G".

### Example 8

### Extrudate/Catalyst Preparation

A mixture of silica (precipitated silica, average particle size 50 µm, surface area 450 m²/g, 75g on a dry basis), monoethanolamine (3.8g as a 5% aqueous solution) acid water (150g) was mulled for a period of 10 minutes. Zirconiom hydroxide (12.2g on basis of ZrO₂) was added and the mixture mulled for a further 5 minutes. Cobalt hydroxide (28.6g on basis of Co₃O₄) was added and the resulting mixture mulled for a further 20 minutes. Finally, polyelectrolyte (Nalco) (2.3g as 2% aqueous solution) was added and the mixture mulled for a further 5 minutes to yield a final mixture having a loss on ignition of 60.7% and a pH of 9.1. The resulting mixture was extruded using a 1" Bonnot extruder having a 1.7 mm Delrin trilobe dieplate insert to yield trilobe extrudates. The extrudates were dried at a temperature of 120 °C and calcined at a temperature of 530 °C for 2 hours. The resulting extrudates, hereafter referred to as catalyst "H", had a high crush strength.

### Example 9

### Extrudate/Catalyst Preparation

A mixture of silica (precipitated silica, average particle size 50 µm, surface area 450 m²/g, 75g on a dry basis), ammonium zirconium carbonate (Bacote) (12.2g on basis of ZrO₂) and water (60g) was mulled for a period of 10 minutes. Acetic acid (4.4g as a 5% aqueous solution) and water (15g) were added and the mulling continued for a further 10 minutes. Water (10g) was added in portions as the mulling was continued for a further 20 minutes. Cobalt hydroxide (28.6g on basis of Co₃O₄) and water (5g) were added and the mixture mulled for a further 10 minutes. Finally, polyelectrolyte (Nalco) (2.3g as a 2% aqueous solution) was added and the mulling continued for a further 5 minutes to yield a final mixture having a loss on ignition of 59.1% and a pH of 8.6. The resulting mixture was extruded using a 1" Bonnot extruder having a 1.7 mm Delrin trilobe dieplate insert to yield trilobe extrudates. The resulting extrudates were dried at a temperature of 120 °C and calcined at a temperature of 530 °C for 2 hours. The resulting extrudates, hereafter referred to as catalyst "I", had a high crush strength.

### Example 10

### Extrudate/Catalyst Preparation

A mixture of silica (precipitated silica, average particle size 50 µm, surface area 450 m²/g, 75 g on a dry basis), monoethanolamine (3.8 g as a 5% aqueous solution) and water (150 g) was mulled for a period of 15 minutes. Titanium dioxide (15 g on a dry basis) was added and the mulling continued for a further 5 minutes. Cobalt hydroxide (33.7 g on basis of Co₃O₄) was added and the resulting mixture mulled for a further 15 minutes. Finally, polyelectrolyte (Nalco) (2.5 g as a 2% aqueous solution) was added and the mulling continued for a further 5 minutes to yield a final mixture having a loss on ignition of 59.2% and a pH of 9.4. The resulting mixture was extruded using a 1" Bonnot extruder having a 1.7 mm Delrin trilobe dieplate insert to yield trilobe extrudates. The resulting extrudates were dried at a temperature of 120 °C and calcined at a temperature of 530 °C for 2 hours. The resulting extrudates, hereafter referred to as catalyst "J", had a high crush strength.
Physical properties of the extrudates and catalysts prepared in comparative Examples 1 to 4, and Examples 5 to 10, were determined and are summarised in Tables I and II.

**TABLE I**

| Extrudates Catalyst | | | | | | | |
|---|---|---|---|---|---|---|---|
| | A | B | C | D | H | I | J |
| Example | 1 | 5 | 5 | 7 | 8 | 9 | 10 |
| Carrier | SiO₂ | SiO₂ | SiO₂ | SiO₂ | SiO₂ | SiO₂ | SiO₂ |
| Base 1) | MEA | MEA | MEA | MEA | MEA | - | MEA |
| Acid 2) | - | - | - | - | - | HAc | - |
| Flow Improver | Nalco | Nalco | Nalco | Nalco | Nalco | Nalco | Nalco |
| Promoter source 3) | - | Zr(OH)₄ | Zr(OH)₄ | - | Zr(OH)₄ | AZC | TiO₂ |
| Co source | - | - | - | Co(OH)₂ | Co(OH)₂ | Co(OH)₂ | Co(OH)₂ |
| Pore volume (H2) (ml/g) | 0.947 | 1.13 | 1.0 | 0.993 | | | |
| Crush strength | high | high | high | high | high | high | high |

| | | | | | | | |
|---|---|---|---|---|---|---|---|
| 1) MEA = monoethanolamine | | | | | | | |
| 2) HAc = acetic acid | | | | | | | |
| 3) AZC = ammonium zirconium carbonate | | | | | | | |

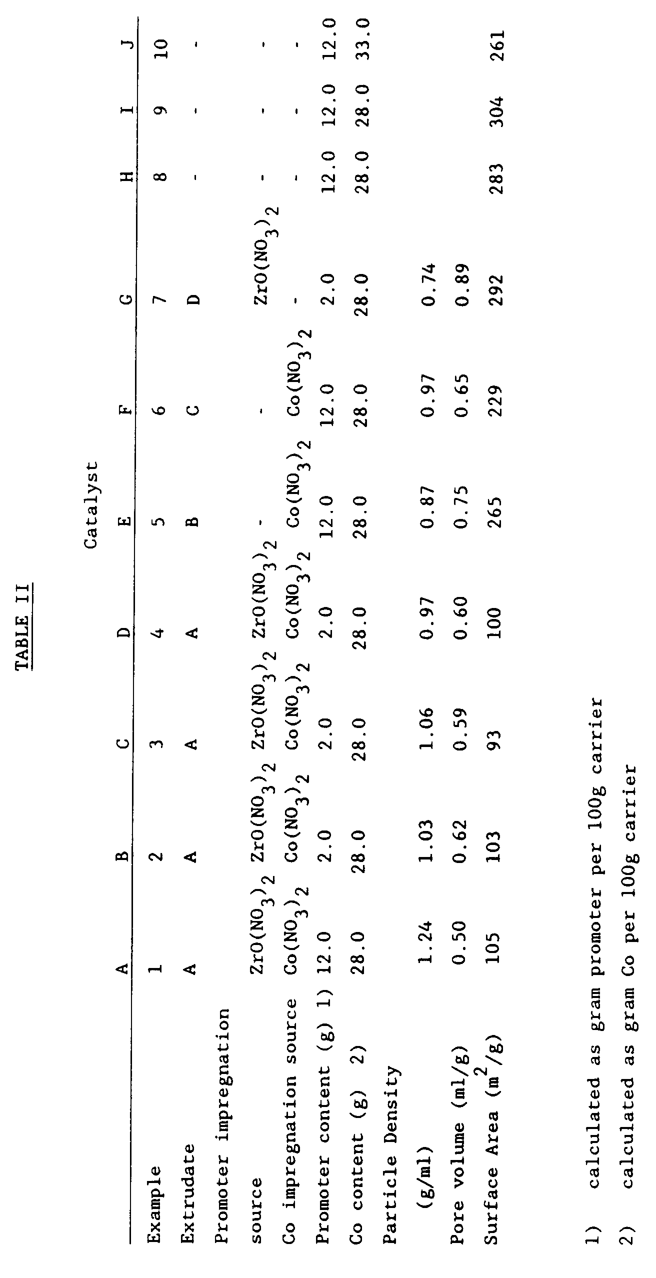

## Claims

1. A process for the preparation of silica-based extrudates for use as a Fischer-Tropsch catalyst or precursor therefor and comprising a catalytically active component selected from elements in Group VIII of the Periodic Table and a promoter selected from elements in Group IVB of the Periodic Table, which process comprises mulling a mixture comprising silica, a solvent and a source of said catalytically active component, which mixture has a solids content of from 20 to 60 %wt; extruding the mulled mixture; and depositing on the resulting extrudates a source of said promoter.

2. A process according to claim 1, further comprising the step of depositing a source of the catalytically active component on the resulting extrudates.

3. A process for the preparation of silica-based extrudates for use as a Fischer-Tropsch catalyst or precursor therefor and comprising a catalytically active component selected from elements in Group VIII of the Periodic Table and a promoter selected from elements in Group IVB of the Periodic Table, which process comprises mulling a mixture comprising silica, a solvent and a source of said promoter, which mixture has a solids content of from 20 to 60 %wt; extruding the mulled mixture; and depositing on the resulting extrudates a source of said catalytically active component; with the proviso that the source of the promoter is insoluble in the solvent.

4. A process according to claim 3, further comprising the step of depositing a source of the promoter on the resulting extrudates.

5. A process for the preparation of silica-based extrudates for use as a Fischer-Tropsch catalyst or precursor therefor and comprising a catalytically active component selected from elements in Group VIII of the Periodic Table and a promoter selected from elements in Group IVB of the Periodic Table, which process comprises mulling a mixture comprising silica, a solvent, a source of said catalytically active component and a source of said promoter, which mixture has a solids content of from 20 to 60 %wt; extruding the mulled mixture.

6. A process according to claim 5, further comprising the steps of depositing a source of the catalytically active component and/or a source of the promoter on the resulting extrudates.

7. A process according to any preceding claim, wherein the catalytically active component is selected from cobalt, iron and nickel, preferably cobalt.

8. A process according to any preceding claim, wherein the promoter is zirconium or titanium, preferably zirconium.

9. A process according to any preceding claim, wherein the silica is a finely divided silica having an ultimate particle size of from 1 to 100 nm, preferably selected from silica gel, precipitated silica or pyrogenic silica.

10. A process according to any preceding claim, wherein the mixture further comprises a base, preferably selected from ammonia, an ammonium compound or an organic base.

11. A process according to claim 10, wherein the base is a promoter source, preferably an ammonium carbonate.

12. A process according to any preceding claim, wherein the pH of the mixture during mulling is in the range of from 8.5 to 11.5.

13. A process according to any preceding claim, wherein the mixture has a solids content of from 20 to 60 %wt, more preferably from 30 to 50 %wt, especially about 40 %wt.

## Patentansprüche

1. Verfahren zur Herstellung von kieselsäurehaltigen Extrudaten zur Verwendung als Fischer-Tropsch-Katalysator oder Vorläufer dafür, enthaltend eine unter den Elementen der Gruppe VIII des Periodensystems ausgewählte katalytisch aktive Komponente und einen unter den Elementen der Gruppe IVB des Periodensystems ausgewählten Promotor, bei dem man eine Kieselsäure, ein Lösungsmittel und eine Quelle der katalytisch aktiven Komponente enthaltende Mischung, die einen Feststoffgehalt von 20 bis 60 Gew.-% aufweist, im Kollergang vermahlt, die gekollerte Mischung extrudiert und auf den erhaltenen Extrudaten eine Promotorquelle abscheidet.

2. Verfahren nach Anspruch 1, bei dem man weiter auf den erhaltenen Extrudaten eine Quelle der katalytisch aktiven Komponente abscheidet.

3. Verfahren zur Herstellung von kieselsäurehaltigen Extrudaten zur Verwendung als Fischer-Tropsch-Katalysator oder Vorläufer dafür, enthaltend eine unter den Elementen der Gruppe VIII des Pariodensystems ausgewählte katalytisch aktive Komponente und einen unter den Elementen der Gruppe IVB des Periodensystems ausgewählten Promotor, bei dem man eine Kieselsäure, ein Lösungsmittel und eine Promotorquelle enthaltende Mischung, die einen Feststoffgehalt von 20 bis 60 Gew.-% aufweist, im Kollergang vermahlt, die gekollerte Mischung extrudiert und auf den erhaltenen Extrudaten eine Quelle der katalytisch aktiven Komponente abscheidet, mit der Maßgabe, daß die Promotorquelle in dem Lösungsmittel unlöslich ist.

4. Verfahren nach Anspruch 3, bei dem man weiter auf den erhaltenen Extrudaten eine Promotorquelle abscheidet.

5. Verfahren zur Herstellung von kieselsäurehaltigen Extrudaten zur Verwendung als Fischer-Tropsch-Katalysator oder Vorläufer dafür, enthaltend eine unter den Elementen der Gruppe VIII des Periodensystems ausgewählte katalytisch aktive Komponente und einen unter den Elementen der Gruppe IVB des Periodensystems ausgewählten Promotor, bei dem man eine Kieselsäure, ein Lösungsmittel, eine Quelle der katalytisch aktiven Komponente und eine Promotorquelle enthaltende Mischung, die einen Feststoffgehalt von 20 bis 60 Gew.-% aufweist, im Kollergang vermahlt und die gekollerte Mischung extrudiert.

6. Verfahren nach Anspruch 5, bei dem man weiter auf den erhaltenen Extrudaten eine Quelle der katalytisch aktiven Komponente und/oder eine Promotorquelle abscheidet.

7. Verfahren nach einem der vorhergehenden Ansprüche, bei dem man als katalytisch aktive Komponente Cobalt, Eisen oder Nickel, vorzugsweise Cobalt, einsetzt.

8. Verfahren nach einem der vorhergehenden Ansprüche, bei dem man als Promotor Zirkonium oder Titan, vorzugsweise Zirkonium, einsetzt.

9. Verfahren nach einem der vorhergehenden Ansprüche, bei dem man als Kieselsäure eine hochdisperse Kieselsäure mit einer Primärteilchengröße von 1 bis 100 nm, vorzugsweise ausgewählt unter Kieselgel, Fällungskieselsäure und pyrogener Kieselsäure, einsetzt.

10. Verfahren nach einem der vorhergehenden Ansprüche, bei dem man der Mischung zusätzlich noch eine Base, vorzugsweise ausgewählt unter Ammoniak, einer Ammoniumverbindung oder einer organischen Base, zusetzt.

11. Verfahren nach Anspruch 10, bei dem man als Base eine Promotorquelle, vorzugsweise Ammoniumcarbonat, einsetzt.

12. Verfahren nach einem der vorhergehenden Ansprüche, bei dem der pH-Wert der Mischung beim Kollern im Bereich von 8,5 bis 11,5 liegt.

13. Verfahren nach einem der vorhergehenden Ansprüche, bei dem die Mischung einen Feststoffgehalt von 20 bis 60 Gew.-%, vorzugsweise von 30 bis 50 Gew.-%, insbesondere von etwa 40 Gew.-%, aufweist.

## Revendications

1. Procédé de préparation d'extrudats à base de silice, à utiliser comme catalyseurs de Fischer-Tropsch ou de précurseurs de ceux-ci et comprenant un composant catalytiquement actif, choisi parmi les éléments du groupe VIII du tableau périodique et un promoteur choisi parmi les éléments du groupe IVB du tableau périodique, caractérisé en ce que l'on broie un mélange constitué de silice, d'un solvant et d'une source du composant catalytiquement actif précité, lequel mélange possède une teneur en solides de 20 à 60% en poids, on extrude le mélange broyé et on dépose une source du promoteur précité sur les extrudats ainsi obtenus.

2. Procédé suivant la revendication 1, caractérisé en ce qu'il comprend en outre l'étape consistant à déposer une source du composant catalytiquement actif sur les extrudats résultants.

3. Procédé de préparation d'extrudats à base de silice à utiliser comme catalyseur de Fischer-Tropsch ou de précurseurs de ceux-ci et comprenant un composant catalytiquement actif choisi parmi les éléments du groupe VIII du tableau périodique et un promoteur choisi parmi les éléments du groupe IVB du tableau périodique, caractérisé en ce que l'on broie un mélange comprenant de la silice, un solvant et une source du promoteur précité, lequel mélange possède une teneur en solides de 20 à 60% en poids; on extrude le mélange broyé et on dépose une source du composant catalytiquement actif précité sur les extrudats ainsi obtenus, avec la condition que la source du promoteur soit insoluble dans le solvant.

4. Procédé suivant la revendication 3, caractérisé en ce qu'il comprend l'étape supplémentaire consistant à déposer une source du promoteur sur les extrudats résultants.

5. Procédé de préparation d'extrudats à base de silice, à utiliser comme catalyseurs de Fischer-Tropsch ou de précurseurs de ceux-ci et comprenant un composant catalytiquement actif, choisi parmi les éléments du groupe VIII du tableau périodique et un promoteur choisi parmi les éléments du groupe IVB du tableau périodique, caractérisé en ce que l'on broie un mélange comprenant de la silice, un solvant, une source du composant catalytiquement actif précité et une source du promoteur précité, lequel mélange possède une teneur en solides de 20 à 60% en poids et on extrude le mélange broyé.

6. Procédé suivant la revendication 5, caractérisé en ce qu'il comprend les étapes supplémentaires consistant à déposer une source du composant catalytiquement actif et/ou une source du promoteur sur les extrudats résultants.

7. Procédé suivant l'une quelconque des revendications précédentes, caractérisé en ce que l'on choisit le composant catalytiquement actif parmi le cobalt, le fer ou le nickel, de préférence, le cobalt.

8. Procédé suivant l'une quelconque des revendications précédentes, caractérisé en ce que le promoteur est du zirconium ou du titane, de préférence, du zirconium.

9. Procédé suivant l'une quelconque des revendications précédentes, caractérisé en ce que la silice est une silice finement divisée possédant un calibre des particules ultime de 1 à 100 nm, de préférence, choisie parmi un gel de silice, une silice précipitée, ou un silice pyrogénée.

10. Procédé suivant l'une quelconque des revendications précédentes, caractérisé en ce que le mélange comprend en outre une base, de préférence, choisie parmi l'ammoniac, un composé d'ammonium, ou une base organique.

11. Procédé suivant la revendication 10, caractérisé en ce que la base est une source de promoteur, de préférence, du carbonate d'ammonium.

12. Procédé suivant l'une quelconque des revendications précédentes, caractérisé en ce que le pH du mélange au cours du broyage varie de 8,5 à 11,5.

13. Procédé suivant l'une quelconque des revendications précédentes, caractérisé en ce que le mélange possède une teneur en solides de 20 à 60% en poids, plus avantageusement, de 30 à 50% en poids, en particulier, d'environ 40% en poids.
